(19) Europäisches Patentamt
European Patent Office
Office européen des brevets

(11) **EP 1 311 719 B1**

(12) **EUROPEAN PATENT SPECIFICATION**

(45) Date of publication and mention
of the grant of the patent:
**06.10.2010 Bulletin 2010/40**

(21) Application number: **01960799.3**

(22) Date of filing: **15.08.2001**

(51) Int Cl.:
***D06M 101/12*** *(2006.01)*

(86) International application number:
**PCT/FI2001/000723**

(87) International publication number:
**WO 2002/014595 (21.02.2002 Gazette 2002/08)**

(54) **A METHOD FOR TREATING PROTEINACEOUS FIBRES**

VERFAHREN ZUR BEHANDLUNG VON PROTEINFASERN

PROCEDE DE TRAITEMENT DE FIBRES PROTEINIQUES

(84) Designated Contracting States:
**AT BE CH CY DE DK ES FI FR GB GR IE IT LI LU MC NL PT SE TR**

(30) Priority: **15.08.2000 FI 20001807**
**15.08.2000 FI 20001808**

(43) Date of publication of application:
**21.05.2003 Bulletin 2003/21**

(73) Proprietor: **VALTION TEKNILLINEN TUTKIMUSKESKUS**
**02044 VTT (FI)**

(72) Inventors:
• **BUCHERT, Johanna**
**FIN-02160 Espoo (FI)**
• **SCHÖNBERG, Christa**
**FIN-13100 Hämeenlinna (FI)**

• **LANTTO, Raija**
**FIN-01800 Klaukkala (FI)**
• **NIKU-PAAVOLA, Marja-Leena**
**FIN-02230 Espoo (FI)**
• **HEINE, Elisabeth**
**50170 Kerpen-Buir (DE)**

(74) Representative: **Sundman, Patrik Christoffer et al**
**Seppo Laine Oy**
**Itämerenkatu 3 B**
**00180 Helsinki (FI)**

(56) References cited:
**EP-A1- 0 852 260     WO-A1-98/05816**
**US-A- 2 539 202     US-A- 5 980 579**

• **DATABASE WPI Week 8625, Derwent Publications Ltd., London, GB; AN 1986-180743, XP002958676 & JP 61 115 488 A (AJINOMOTO KK) 06 June 1986**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

This invention relates to a novel method for treating proteinaceous fibres according to the preamble of claim 1 and to a product produced by the method according to the preamble of claim 27.

## Background of the invention

Wool is an extremely complex, highly crosslinked protein fibre. Examples of other protein fibres are human hair, alpacca, mohair, cashmere, angora, lama and silk. Wool protein is keratin. Pure wool is composed of 97 % protein, 2 % lipids and 1 % other compounds, such as salts and carbohydrates. A large part of the keratin is formed of microcrystalline $\alpha$-helix structures. A significant amount of either inter- or intra-chain covalent S-S-crosslinks exists in keratin due to the high amounts of cysteine. Cleavage of these bonds or their rearrangement is achieved in many important wool processes, such as shrink-proofing or setting. In addition to covalent crosslinks hydrogen bonds, ionic bonds and hydrophobic interactions exist in wool.

Wool shrinkage is a typical property of animal, keratin fibres (Makinson, 1979). Shrinkage occurs during wool processing steps, which include scouring, carding, spinning, weaving, piece dyeing and finishing. According to Makinson (1979) the shrinkage of wool can be classified into three distinct classes: 1) hygral expansion, which depends on the water content of the fibres, 2) relaxation shrinkage, which is caused by the rearrangement of hydrogen bonds formed during processing as wool is immersed in water after processing and 3) felting shrinkage, which is caused by the rootward migration of the fibres. Hygral expansion and relaxation shrinkage are properties which basically cannot be affected whereas felting shrinkage can. Felting shrinkage requires agitation and is progressive. Prevention of felting shrinkage is crucial for shrink-proofing of wool and is carried out by preventing the rootward migration of the fibres.

Shrink-proofing of wool has been carried out mainly by using two distinct processes; the chlorine/Hercosett-process and the Dylan GRC-process (Makinson, 1979; Lewis, 1992). Both processes consist of a chlorinating and dechlorinating step followed by addition of a polymer. Chemical degradation of the scales by chlorine results in increased amounts of charged groups in the fibre while the disulphide bridges are oxidised to cysteic acid groups (Makinson, 1979). Polymer treatments aim at softening the scales by burying the scales beneath a polymer film (Makinson, 1979). In addition to these processes several other processes have been developed such as SIMPL-X (EP 618 986). There are also two processes which feature only a polymer application i.e. Synthaprett BAP (Bayer) and DC 109 (Dow Corning/PPT) (Byrne, 1995). The current shrink-proofing processes have several drawbacks e.g. chlorine treatments result in generation of AOX-compounds, which are harmful to the environment.

Research on enzymatic wool processing was started with proteinases hydrolysing peptide linkages. The use of proteinases for wool processing has been extensively studied in the literature but proteinase treatments have resulted in high loss of strength and weight losses as reviewed by Ellis (1995).

Enzymatic methods for wool processing have been described for example in the following patent publications: WO 96/19611 describes the use of proteinases to modify the scales of the wool fibres to confer resistance to felting shrinkage. WO 98/27264 describes a method for reducing the shrinkage of wool comprising contacting wool with an oxidase or a peroxidase solution under conditions suitable for reacting the enzyme with wool. US 5,529,928 describes a process for obtaining a wool with a soft woolly handle and shrink-resistant properties by using an initial chemical step or an enzyme treatment (e.g. a peroxidase, a catalase, or a lipase) followed by a proteinase and heat treatments. EP 358386 describes a method to treat wool which comprises a proteolytic treatment and either or both of an oxidative treatment (such as NaOCl) and a polymer treatment. EP 134267 describes a method for treating animal fibers with an oxidizing agent, followed by a proteolytic enzyme in a salt-containing composition. WO 99/60200 describes the treatment of wool, wool fibres or animal hair with a proteolytic enzyme and a transglutaminase.

Reports on the use of laccases, which belong to phenoloxidases, to increase the shrink resistance of wool have been published (WO 94/25574). Laccase treatments were carried out either with HBT- or ABTS-mediators. The treated wool flannels were reported to be less subjective to shrinkage after the treatments. The chemical changes caused by the laccase treatments were not analysed. A method for reducing shrinkage of wool by contacting a wool containing article with oxidase or peroxidase, and in particular laccase from *Trametes* spp. or *Pleurotus* spp. is described in WO 98/27264. However, with the powerful radical forming mediators HBT and ABTS the wool fibre may be over-oxidized and damaged. Furthermore staining of wool is occurring in for instance laccase-HBT reaction. This staining has a negative impact on further dyeing stages of wool.

WO 98/05816 describes an enzymatic method for overdyeing cellulosic textiles. In the method a fabric or article is treated in an aqueous dye system which comprises mono-, di-or polycyclic aromatic or heteroaromatic compounds and a hydrogen peroxide source and peroxidases and/or oxidases. WO 00/31333 describes a method for dyeing a material by treating the material with a dyeing system which comprises reduced vat dyes and/or reduced sulfur dyes and these reduced dyes are oxidized with an oxidation system which comprises an oxygen source and oxidases or a hydrogen peroxide source and peroxidases. WO 99/15137 relates to enzymatic foam compositions adapted for dyeing of kerationous fibres, which comprises oxidation enzyme, foaming agent, dye precursor and optionally a modifyer. All these three patent publications describe a method, where the enzyme used in the method reacts with a dye or with a dye precursor, but not directly with fibres. US patent 6,140,109 describes treatment of wool with a haloperoxidase together with a hydrogen peroxide source and a halide source.

Although significant progress has been achieved with the use of chemicals in the wool industry to prevent shrinkage, the drawback remains that many of the chemical processes are environmentally harmful. On the other hand many of the enzymatic methods result in damages to wool by causing weight and strength losses. Thus an improved enzymatic method to treat wool, wool fibres, or animal hair material which imparts improvements in shrink-resistance and other properties, but causes less fibre damage than known enzymatic treatments, is still needed.

## SUMMARY

It is an aim of the present invention to eliminate the problems associated with the prior art and to provide a solution to the problems in the methods of treating wool or wool-containing materials or other protein originating or containing material. The present invention provides in particular an improved method for treating proteinaceous fibres. The method comprises contacting proteinaceous material with an aqueous solution comprising tyrosinase enzyme under suitable conditions for reacting tyrosinase with the material.

The method is defined in claim 1.

The method of this invention can be applied to treat protein containing fibres, for instance keratin fibres. It is suitable to treat wool, wool fibre or animal hair, such as angora, mohair, cashmere, alpacca, or other commercially useful animal hair product, which may originate from sheep, goat, lama, camel, rabbit etc. Also silk, spidersilk or human hair can be treated with the method of this invention. The fibres may be in the form of fibre, top, yarn or woven or knitted fabric or garments.

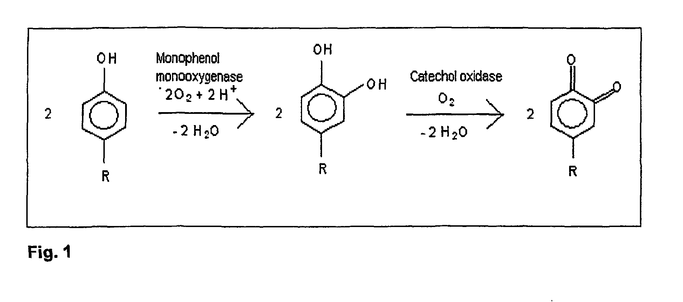

The tyrosinase enzyme may originate from any plant, animal or microbial origin capable of producing tyrosinase or the enzyme may originate from a new source or it may be produced by genetic engineering methods in a suitable host organism. The enzyme is capable of catalyzing the conversion of tyrosine residues in proteinaceous material to corresponding hydroxyls or alternatively first to hydroxyls and subsequently to quinones(see Figure 1).

Preferably the enzyme originates from berries, fruits (citrus fruits, such as grape, apple, pear, peach, banana), vegetables (potato, cabbage, pea, bean, cucumber, tomato, spinach, olive), cereals (barley, wheat, rye), tea, coffee- and cacao-beans, animals (mouse, frog, shrimps), edible mushrooms (*Agaricus*, such as *Agaricus bisporus, Pleurotus, Lentinus*), fungi (*Neurospora crassa, Aspergillus,* such as *Aspergillus oryzae*), bacteria (*Pseudomonas*, such as *P. maltophilia, Xanthomonas,* such as *X. maltiphilia Stenotrophomonas,* such as *S. maltophilia, Streptomyces,* such as *S. glaucescens, S. antibioticus, S. castaneoglobisporus, Vibrio,* such as *Vibrio tyrosinaticus, Rosebacterium, Thermomicrobium,* such as *Thermomicrobium roseum, Marinomonas,* such as *M. mediterranea, Alternaria,* such as *Alternaria tenuis, Alteromonas* or *Rhizobium*).

According to a preferred embodiment the enzyme orginates from potato or_from bacteria belonging to *Pseudomonadaceae,* such as from *Pseudomonas,* or from bacteria, which are closely related to *Pseudomonas,* such as from *Xanthomonas,* which was earlier classified to *Pseudomonas* or from *Stenotrophomonas,* which was earlier classified to *Xanthomonas*. In particular, tyrosinase may originate from micro-organism strains belonging to the genus or species represented by the strain deposited in connection of this invention in DSMZ-Deutsche Sammlung von Mikroorganismen und Zellkulturen GmbH on 16 June 2000 under number DSM 13540. The strain was identified to belong to *Pseudomonadaceae,* genus *Stenotrophomonas,* species *Stenotrophomonas* sp. although the strain was also closely related to genus *Pseudomonas,* species *Pseudomonas beteli.*

Tyrosinase may originate also from microorganisms known to produce laccases, for example from *Trametes,* such as *T. hirsuta* or *T. versicolor* or from *Myceliophthora.*

The treatment dosages of tyrosinase per kg wool, wool-containing material, or other proteinaceous fibre containing material is 1 nkat/g - 5000 nkat/g of protein fibre material or 10 mg/kg - 50 g/kg of protein fibre material. More preferably the treatment dosages are 10 nkat/g - 500 nkat/g or 100 mg/kg to 5 g/kg. The treatment time can be 5 minutes - 24 h, more preferable 30 min to 2h. The treatment can be carried out on different stages of processing. The treatment may be carried out before, during and/or under shrinkage conditions and/or before and/ or during dyeing or other finishing stages.

The treatment may be carried out at pH range pH 3-8, preferably pH 5-7. The processing temperature may be 20 - 80 °C, preferably 30-70 °C, most preferably 40 -50 °C.

The tyrosinase treatment may be combined with an accelerator or mediator enhancing the enzyme activity of tyrosinase. The tyrosinase treatment may also be combined to a reducing agent such as ascorbic acid.

Furthermore proteinaceous fibre may be treated before, during or after the tyrosinase treatment with another enzymatic or a chemical treatment. Enzymatic treatments may comprise for example proteinase, lipase, lipoxygenase, laccase, peroxidase, haloperoxidase, transglutaminase or protein disulphide isomerase treatment or any of their combination. Chemical treatments may comprise any chemical treatments used conventionally for shrink reduction. Such chemical treatments are for example treatment with chlorine-based shrink reducing agent.

The tyrosinase treatment may be combined also with chemical additives such as wetting agents and softeners.

Preferably the tyrosinase treatment of this invention is carried out before or during a process step with or without agitation.

This invention provides also protein originating fibres, which are treated with the method of this invention.

The fibres may be wool, wool fibre or animal hair, such as angora, mohair, cashmere, alpacca, or other commercially useful animal hair product, which may originate from sheep, goat, lama, camel, rabbit etc. The fibres may be in the form of fibre, top, yarn or woven or knitted fabric or garments. One object of this invention are also human hair, silk or spider silk treated according to the method of this invention.

According to one preferred embodiment of this invention the proteinaceous material is treated with a novel enzyme, which is isolatable from a micro-organism strain, which belongs to the genus or species represented by the strain deposited in connection of this invention and having the accession number DSM 13540. The strain is identified to belong to *Stenotrophomonas* sp. The pH-optimum of the enzyme is 8.0 and the temperature-optimum 40-50 °C. The molecular weight estimated by SDS-PAGE is about 95 000 Da (or 95 +/- 1 kDa) and the pI-value estimated by isoelectric focusing was about 5 (or 5+/-0.5). The enzyme is especially suitable for treating proteinaceous materials, particularly wool. By crosslinking proteins tyrosinase can stabilize the structure of wool protein and thus prevent scales of wool fibre to slide rootward in felting conditions. The enzyme consumes oxygen i.e. oxidises wool protein in the conditions favourable to wool treatment at pH 5 - 7 and in the temperature 40-50°C. Oxidation of tyrosine residues of wool fibres by the enzyme increased the oxidation level of carbon present on the fibre surface as measured by ESCA.

The tyrosinase treatment can result in several benefits depending on the processing method used. The treatment can result in more crosslinking, leading to greater strength, better creasing behaviour and a reduction in felting shrinkage. Furthermore the wettability may be altered due to surface oxidation thus improving the dyeing or printing of the fabric. Formation of reactive sites on the fibre can result in improved dyeing behaviour.

Chemical oxidation of wool leads to oxidation of sulphur crosslinks and as a result the fibre structure is weakened and the fibre is rendered more susceptible to mechanical and chemical damage. On the other hand oxidation of amino acid moieties present in wool structure by tyrosinase leads to other types of oxidations thus not necessarily affecting sulphur crosslinks. As a result the fibre strength can be retained more efficiently.

One benefit in addition to those listed above is the novel environmentally friendly process for wool or other protein containing animal fibre with special emphasis on shrink-resistant treatment which provides the flexibility and low pollution characteristics demanded by the global textile market. It also imparts machine washability, improves dyeability and comfort factor of wool or other protein containing animal fibre apparel. Due to the functionality of quinones formed from tyrosine residues different types of chemicals can potentially be grafted to wool fibres via these sites.

**Deposits**

The microorganism strain isolated in this invention was deposited according to the Budapest Treaty on 16 June 2000 at the DSMZ- Deutsche Sammlung von Mikroorganismen und Zellkulturen GmbH, Mascheroder Weg 1 b, D-38124 Braunschweig, Deutschland, and assigned as DSM 13540. The strain was identified (ID 00-309 ) to belong to *Stenotrophomonas* sp.

**BRIEF DESCRIPTION OF THE FIGURES**

Figure 1 shows the reactions catalysed by tyrosinase.

Figure 2 depicts tyrosinase activity, pH and amount of viable cells during cultivation of the tyrosinase producing bacterium DSM 13540. cfu = colony forming units.

Figure 3A shows pH- and Figure 3B shows temperature-optimum of the partially purified tyrosinase activity produced by DSM 13540 isolated in this invention. The substrate was 2 mM Dopa, incubation 10 min at 50 °C, A475nm.

Figure 4 depicts the reactivity of different tyrosinases with wool fibres as measured by oxygen consumption.

Figure 5 depicts the reactivity of tyrosinase produced by DSM 13540 with wool fibres as measured by oxygen consumption.

Figure 6 shows the alkaline solubility of tyrosinase treated samples.

**DETAILED DESCRIPTION OF THE INVENTION**

The term "proteinaceous fibre" denotes here any protein originating fibres, which comprise keratin fibre structure or is intended to be woven or processed into a textile garment or proteinaceous fibre containing article, e.g., carpets, hats etc.. The term comprises wool, wool fibre or animal hair or other commercially useful animal hair product, which may originate from sheep, goat, lama, camel, rabbit etc. Examples are angora, mohair, cashmere, alpaca, merino and Shetland wool. The term comprises also silk, spider silk and human hair. The fibres may be in the form of fibre, yarn or woven or knitted fabric or garments. Proteinaceous material which may be processed according to the present invention includes compositions which comprise other non-proteinaceous components for example blends of wool (or other proteinaceous fibre) with cellulosic fibres (cotton, linen, ramie, jute, sisal, etc) or blends of wool (or other proteinaceous fibre) with synthetic fibres such as polyamide, polyester, polypropylene, acrylic, spandex, nylon or with man-made fibres, such as viscose, rayon, acetate, Lyocell®, Tencel®, etc. The compositions of this invention comprise at least 20 %, more preferably at least 50 %, most preferably at least 80 % proteinaceous materials.

By the expression "oxidation of tyrosine residues in proteinaceous fibre" is meant here that the tyrosine residues in proteinaceous fibre are oxidised by a measurable amount as compared to nontreated proteinaceous fibres. The oxidation can be measured by e.g. ESCA or Raman spectroscopy. The tyrosinase treatment results in oxidation, which is indicated by increase in the O/C ratio at least by 5 %, preferably at least by 7 %, most preferably at least by 10 % in treated proteinaceous fibres as compared to nontreated proteinaceous fibres. The fibres are oxidised either only by hydroxylation or alternatively both hydroxylation and subsequent quinone formation. If the modification of tyrosine residues is measured by Raman spectroscopy the change in adsorbancy is at least 5 %, preferably at least 10 %, most preferably at least 20 %.

The term "shrinking" denotes here the ability of proteinaceous material such as wool to shrink when processed under shrinking conditions.

"Shrinking conditions" denotes here conditions under which proteinaceous material such as wool tends to irreversibly shrink by a felting mechanism. Conditions causing felting shrinking are described in Makinson (1979). The presence of water specifically in connection with mechanical agitation is known to cause shrinking. Other conditions reported to increase felting alone or in combinations are temperatures between 20 to 60°C or higher, acid or alkali, presence of detergents (soap) in alkaline or neutral media, the presence of neutral salts, lubricants, alcohol and agitation.

"A reduction in felting shrinkage" denotes here decrease of shrinkage when compared to shrinkage without the treatment of this invention.

"Conventional shrink resist processes and chemical agents" denotes here processes and chemical agents used presently by industry to control or reduce felting shrinkage. Such processes may be carried out on a batch or continuous basis, and generally comprise multistep treatments, involving oxidation of the wool, followed by the application of a polymer. The most commonly used oxidising agents are chlorine and chlorinating agents or peroxygen compounds, such as permonosulphuric acid. Polymers include the widely used Hercosett polymer (polyaminoamide) or one of a number of specially developed polymers. In current commercial application, the only alternatives to these oxidation-polymer processes are the polymer-only processes, whereby a polymer is deposited and cured on the surface of the textile. These processes have a more limited application, and are only applicable to manufactured textile articles.

The term "handle" refers to the subjective reaction obtained by feeling a fabric and assessing its roughness, harshness, flexibility or softness.

The term "softness" refers to the feel of a textile that is flexible with a surface which is easily deformed and a pleasant touch.

### Tyrosinase enzymes

Tyrosinase belongs to phenoloxidases, which use oxygen as cosubstrate and is thus particularly suitable for enzymatic processes as no valuable cofactors such as NAD(P)H/NAD(P) are required in the reactions. Tyrosinase catalyses both the *o*-hydroxylation of monophenols and the oxidation of *o*-diphenols to *o*-quinones (EC 1.14.18.1; monophenol monooxygenase, EC 1.10.3.1; catechol oxidase) (Lerch, 1981). Therefore, the differentiation of the enzymatic activity into two distinct reactions only distinguishes between two reactions catalysed by the same enzyme (Mayer, 1987). As wool contains significant amount of tyrosine residues and their amount is dependent on the location of the protein in wool fibre, whether it is from cuticle, cortex, resistant membranes or intracellular cement in the fibre. Whole wool contains about 3-4% of tyrosine whereas intercellular cement contains over 7 % of tyrosine. Thus, tyrosinases are especially useful to modify the properties of wool or other protein fibres.

Tyrosinases can be distinguished from laccases although both use molecular oxygen to oxidise quite similar substrates. A typical property of tyrosinase is inability to catalyse the oxidation of p-diphenols (Mayer and Harel, 1979). On the other hand, failure to oxidise tyrosine but ability to oxidise L- dihydroxyphenylalanine (L-DOPA) can be taken as proof of laccase activity (Mayer, 1987).

The primary structures of tyrosinases from *Rhizobium meliloti* (Mercado-Blanco et al., 1993), *Streptomyces* (Bernan et al., 1985; Huber et al., 1985; Kawamoto et al., 1993; Ikeda et al., 1996), *Aspergillus oryzae* (Fujita et al., 1995), *Neurospora crassa* (Kupper at al., 1989), *Rana nigromaculata* (Takase et al., 1992), *Mus musculus* (Kwon et al., 1988; Muller et al., 1988) and *Homo sapiens* (Kwon et al., 1987; Giebel et al., 1991) have been analysed and have considerable homogeneity. In the catalytic domain of all these enzymes there is a single binuclear copper center similar to that of hemocyanin (Gaykema et al., 1991).

The ability of tyrosinase to crosslink food proteins has been reviewed (Matheis and Whitaker, 1987). According to Matheis and Whitaker (1984a) the crosslinking of proteins with tyrosinase proceeds via the formation of *o*-quinones from tyrosine. These *o*-quinones either condense with each other or react with protein amino and sulfhydryl groups in proteins (Matheis and Whitaker, 1984a). Tyrosinase is also able to catalyse the oxidation of the *p*-hydroxyphenyl group of tyrosine residues also resulting in crosslinking of proteins (Matheis and Whitaker, 1984b; EP 947 142).

Addition of a low-molecular-weight phenolic compound, such as tyrosine or tyrosine containing polypeptides or other non-phenolic mediators can increase the ability of tyrosinase to crosslink or modify proteins. In the case of using tyrosine as mediator, the enzymatically generated *o*-quinones can react with the amino, sulfhydryl, thioether, phenolic, indole and imidazole groups present in proteins (Matheis and Whitaker, 1984a; Matheis and Whitaker, 1984b).

Tyrosinase has been used to polymerise tropocollagen macromolecules, which are the constituents of collagen fibres (Dabbous, 1966). Formation of inter- and intramolecular crosslinks between tyrosine residues resulted in polymerisation.

Novel tyrosinases suitable for modification of proteinaceous material can be searched from microbes isolated from process or natural environments containing appropriate protein as substrate for the tyrosinase activity. Microbes are screened for tyrosinase activity by cultivating them on suitable screening media and isolated as pure cultures. Microbes are cultivated in suitable liquid media inducing tyrosinase production. Tyrosinases can also be isolated from berries, fruits (grape, apple, pear, peach, banana), vegetables (potato, cabbage, pea, bean, cucumber, tomato, spinach, olive), cereals (barley, wheat, rye), tea, coffee- and cacao-beans, animals (mouse, frog, shrimps) and edible mushrooms (*Agaricus,* such as *Agaricus bisporus, Pleurotus, Lentinus*), fungi (*Neurospora crassa, Aspergillus,* such as *Aspergillus oryzae),* bacteria, from *Pseudomonas,* such as *P. maltophilia, Xanthomonas,* such as *X. maltiphilia Stenotrophomonas,* such as *S. maltophilia* or other bacteria belonging to *Pseudomonadaceae, Streptomyces,* such as *S. glaucescens, S. antibioticus, S. castaneoglobisporus, Vibrio,* such as *Vibrio tyrosinaticus, Rosebacterium, Thermomicrobium,* such as *Thermomicrobium roseum, Marinomonas* such as *M. mediterranea, Alternaria,* such as *Alternaria tenuis, Alteromonas* or *Rhizobium).* Tyrosinase may be isolated also from microorganisms known to produce laccases, for example from *Trametes,* such as *T. hirsuta, T. versicolor* or from *Myceliophthora.*

Tyrosinases having necessary pH and temperature characteristics are let to react with wool or other proteinaceous material. Tyrosinases can be applied as culture filtrates or as partially or completely purified proteins. The tyrosinase encoding gene can be cloned from any organism mentioned above and subsequently transferred to an appropriate production organism. Suitable expression and production hosts are for example fungi such as *Trichoderma* and *Aspergillus,* yeast, bacteria such as *Bacillus* and *E. coli.* Screening of efficient tyrosinases can be carried out by measuring the oxygen consumption during the enzymatic reaction.

**Fibre, yarn or fabric treatment conditions**

The treatment dosages of tyrosinase per kg of proteinaceous fibres in any form, preferably wool or wool-containing material, is 1 nkat/g - 5000 nkat/g of protein fibre material or 10 mg/kg - 50 g/kg of protein fibre material. More preferably the treatment dosages are 10 nkat/g - 500 nkat/g or 100 mg/kg to 5 g/kg. The treatment time can be 5 minutes - 24 h, more preferable 30 min to 2h. The treatment may be carried out at pH range pH 3-8, preferably at pH range 5-7. The processing temperature maybe 20 - 80 °C, preferably 30-70 °C, most preferably 40 -50 °C. The treatment can be carried out on different stages of processing. The treatment may be carried out before, during and/or under shrink resist processes and/or before and/ or during dyeing or other finishing stages. The treatment may also be carried out as such without any other chemical step.

The enzyme process can potentially be carried out at any stage of wool processing - as with the chemical shrink resist treatments - from fibre through to knitted or woven articles. Similarly, the enzyme process could be carried out before or after dyeing. The enzymatic treatment can also be coupled to a chemical treatment, such as oxidation either by preceding or following the enzymatic stage. The enzyme stage can also be carried out in combination with a reducing agent, such as ascorbic acid. One possibility is to conduct a chemical and enzyme treatment concurrently provided that the conditions of the chemical treatments are compatible with the enzyme process. The treatments can be carried out in different types of textile machinery, such as in a side paddle or a rotary machine when knitted fabrics are treated or in winches, jets or jigs when woven fabrics are treated.

The following examples are for illustration of the present invention and should not be construed as limiting the present invention in any manner.

## EXAMPLES

### Example 1. Screening for tyrosinases from micro-organisms isolated from a wool processing mill

Dirt, wool and dust samples from a wool processing mill were collected from different stages of the process. In order to isolate micro-organisms 1 ml of the liquid samples were diluted to 10 ml with physiological salt solution (0.9 % w/v NaCl-solution). Approximately 1 g of the solid samples were immersed in 10 ml physiological salt solution whereafter all samples were agitated at 200 rpm for 2 hours. After the agitation dilutions were prepared (generally $10^{-2}$-$10^{-4}$). Bacterial strains were isolated from the dilutions by cultivating samples on Plate Count Agar-plates (PCA-plates) containing 0.01 % Benomyl (Sigma) in order to inhibit fungal growth. Fungal strains were isolated using Malt Extract Agar-plates (MEA-plates) containing 0.01 % chloramphenicol and chlortetracycline (Sigma) for inhibition of bacterial growth. All micro-organisms were grown at 30 °C. After the first cultivations pure cultures were prepared by sequentially inoculating individual bacterial colonies or fungal spores to PCA- or MEA-plates, respectively.

Different colour reagents were used as indicators of oxidative enzyme activity on plate cultures (Table 1). Tyrosine (Sigma) was used as the tyrosinase specific indicator. The laccase specific indicator used was guaiacol (Merck). In addition, Remazol Brilliant Blue R (RBBR, Sigma) was used as indicator for oxidative enzyme activity. Guaiacol and RBBR were used as described previously in Paasivirta (2000).

Table 1. Specific indicators used in the screening, their colour reactions and concentration on plates.

| Indicator | Concentration % | Colour change during oxidation |
|---|---|---|
| Tyrosine | 1.0% | Colourless, white → brown |
| Guaiacol | 0.01 % | Colourless → purple red |
| RBBR | 0.02% | Blue → colourless |
| RBBR = Remazol Brilliant Blue R | | |

Approximately 30 fungi and 95 bacteria were isolated from a wool processing mill to pure cultures and screened for tyrosinase, laccase and peroxidase activities. Of these screened micro-organisms none were found to be laccase- and peroxidase-positive, but eight bacteria were found to be tyrosinase-positive (Table 2 ).

Table 2. Origin of tyrosinase-positive bacteria isolated from a wool processing mill.

| Bacterium code | Origin |
|---|---|
| BW 30 | process waste |
| BW 41 | carding waste |
| BW 61 | gilling waste |
| BW 64 | wall |
| BW 65 | wall |
| BW 68 | wool waste |
| BW 88 | floor |
| BW 90 | carding waste |

BW 65 was the bacterial strain isolated in this invention and deposited in DSMZ-Deutsche Sammlung von Mikroorganismen und Zellkulturen GmbH on 16 June 2000 under number DSM 13540. The deposited strain was identified by DSMZ to be *Stenotrophomonas* sp. Identification of the strain to *Stenotrophomonas maltophila, Pseudomonas beteli, Pseudomonas hibiscicola* and *Pseudomonas geniculata* had according to the identification of DSMZ been also possible.

### Example 2. Production of tyrosinase by DSM 13540

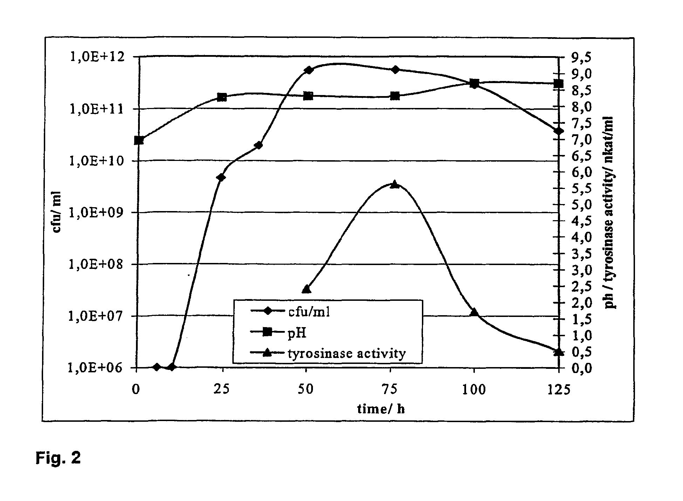

The deposited strain DSM 13540 was cultivated using medium containing 20 g/l glucose, 2.5 g/l casein, 2 g/l tyrosine and 2 g/l wool. Peroxidase, laccase, neutral proteinase and tyrosinase enzyme activities were measured during the cultivation. Additionally, the amount of viable cells and the pH of the culture liquid were measured. Tyrosinase activity reached its maximum as the cells reached their lag phase (Figure 2). The highest tyrosinase activity was approximately 8 nkat/ml. Neither laccase nor peroxidase activities were detected in the culture liquid.

Tyrosinase activity was determined using 2 mM DL-DOPA (Sigma) as substrate. The pH of the substrate solution was adjusted to 7 with 0.05 M sodiumphosphate buffer. 40 μl of the sample was mixed with 960 μl 2 mM DL-DOPA solution. Tyrosinase activity was determined by measuring the increase in absorbance at 475 nm for three minutes at 30 °C with a Perkin Elmer Lambda 20-spectrophotometer. The activity was calculated according to the formula.

$$tyrosinase\ activity\ (nkat/ml) = \frac{10^6 \times \Delta Abs_{475nm} \times V_{tot}}{V_n \times t \times \varepsilon \times k \times l}$$

where,

$\Delta Abs_{475nm}$ is the increase in absorbance in the reaction time at 475 nm
$V_{tot}$ is the total volume (1 ml)
$\varepsilon$ is the molar extinction coefficient of the oxidised dopachrome (3400 lmol$^{-1}$cm$^{-1}$)
t is the reaction time (s)
$V_n$ is the volume of the sample (0.04 ml)
k is the dilution coefficient
1 is the distance light travels in the cuvette (1 cm)

One unit (katal) of tyrosinase activity is defined as the amount of enzyme that catalyses the formation of 1 mole of dopachrome per second at 30 °C and pH 7.0.

Samples of 1 ml were taken at different time intervals during the cultivations. Proteinase activity was inhibited by adding 0.1 ml of 17 mg/ml phenylmethylsulphonyl fluoride (PMSF, Sigma) solution to the samples. The supernatant was cleared by centrifugation at 4°C and tyrosinase activity was measured from the supernatant.

**Example 3. Isolation of potato tyrosinase**

Potato tyrosinase was isolated from a potato variety called Asterix. Peeled potatoes, 500 g fresh weight, 95 g dry weight, were homogenized with 200ml 0.1M Na-phosphate buffer pH 7.0 using Waring Blendor homogenizer for 4x 15 s at high power. The suspension was further stirred at 750 rpm in ice bath for 30 min and filtered through bolting cloth and glass fibre filter (Whatman GF/C). The final filtrate volume was 300ml. Phenols inactivating tyrosinase activity in the filtrate were separated from proteins by precipitating the proteins with 70% ammonium sulphate saturation for 1 h at 4°C. The precipitate was dissolved in 250 ml of 0.1M Na-phosphate buffer pH 7.0. The crude potato tyrosinase was stored at 4°C (Table 3).

Table 3 Preparation of crude potato tyrosinase

| Step | Activity nkat/ml | Volume ml | Total activity Nkat | Activity yield % | Protein mg/ml | Total protein Mg |
|---|---|---|---|---|---|---|
| Extract | 20 | 300 | 6000 | 100 | | |
| Crude preparation | 16 | 250 | 4000 | 67 | 1.5 | 375 |
| Crude potato tyrosinase did not contain proteolytic impurities. | | | | | | |

**Example 4. Determination of pH- and temperature-optimum of the novel tyrosinase**

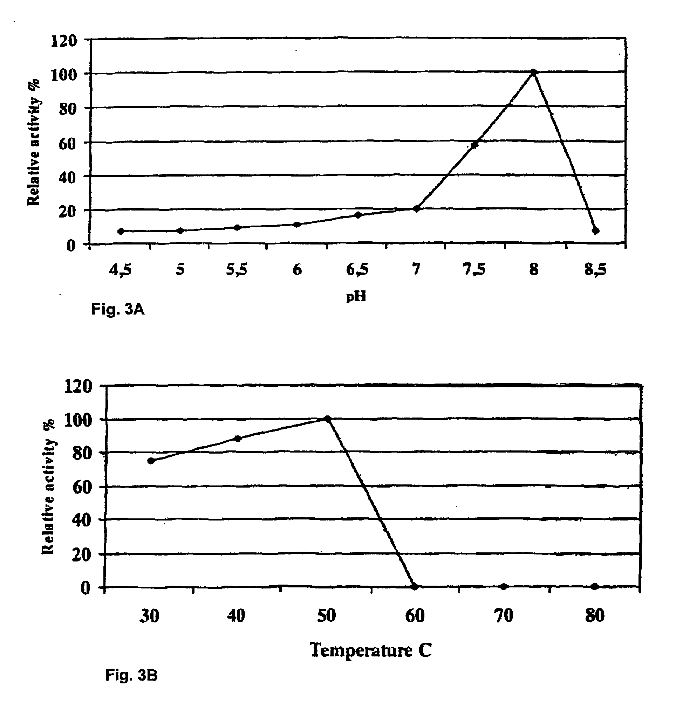

The novel tyrosinase produced by DSM 13540 strain was partially purified from the concentrated culture filtrate by ion exchange chromatography. The culture filtrate was concentrated by ultrafiltration and the concentrate was adsorbed on Q-sepharose anion exchanger equilibrated with 25 mM phosphate buffer pH 7. The enzyme was eluted with a gradient consisting of the starting buffer and the starting buffer supplied with 0.3M NaCl. Tyrosinase and proteolytic activities of the fractions were monitored. Fractions containing only tyrosinase activity were pooled and used for characterization of the novel enzyme. Purification yielded 58% of the acitivity in the culture concentrate. The pH- and temperature optimum were determined by measuring the tyrosinase activity of the partially purified enzyme preparation at pH 6.5...9.0 DL-DOPA (2 mM) was used as substrate and the reaction mixture was incubated for 10 minutes whereafter the absorbance at 475 nm was recorded. The temperature-optimum of the novel tyrosinase was determined by measuring the tyrosinase activity at 20 °C...90 °C at the pH-optimum. The pH-optimum was found to be 8.0 and the temperature-optimum 40-50 °C (Figure 3A and 3B).

The tyrosinase enzyme has been described also in our copending international patent application, the content of which is hereby fully incorporated by reference. The international application is filed on the same day as the present application and is based on Finnish patent application No 20001808 filed on 15 August 2000.

**Example 5. Determination of molecular weight and isolelectric point of the novel tyrosinase**

The biochemical properties of the novel tyrosinase were characterized by standard techniques. The molecular weight was estimated by SDS-PAGE and the pI-value with isoelectric focusing. The MW was about 95 000 Da and the pI-value about 5.

**Example 6. Determination of substrate specificity of the novel enzyme**

The substrate specificity of the novel enzyme was deteermined by using 2mM tyrosine, catechol and DOPA as substrates. The relative activites towards these substrates at pH 8 and 50 °C were 10, 40 and 100 %, respectively.

**Example 7. Isolation of the gene encoding the novel tyrosinase**

From the purified tyrosinase protein N-terminal and internal peptide sequences are sequenced. Based on peptide sequences oligonucleotide probes are constructed by common PCR techniques. Oligonucleotide probes are used in cloning the tyrosinase encoding gene from DSM13540 genome.

**Example 8. Reactivity of tyrosinases with wool fibres as measured by oxygen consumption**

The ability of different tyrosinases, *i.e. Agaricus bisoprus* (Sigma), potato tyrosinase (from Example 3) and DSM 13540 tyrosinase (from Example 2), to oxidise wool fibres were elucidated by measuring the oxygen consumption of the enzyme reactions in sealed Erlenmeyer flasks with an Orion Research 97-08 oxygen electrode according to the VTT Biotechnology Standard 5555-95. The tyrosinase dosage used was 500 or 1000 nkat/g wool fibres. The scoured wool fibres were used as substrates. The treatments were carried out at 45 °C with 200 rpm agitation. The wool to liquid ratio used was 1:100. The concentration of the dissolved oxygen was measured for 45 minutes every 15 seconds.

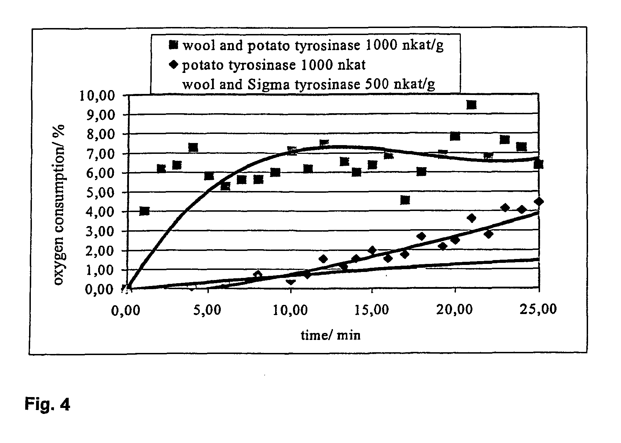

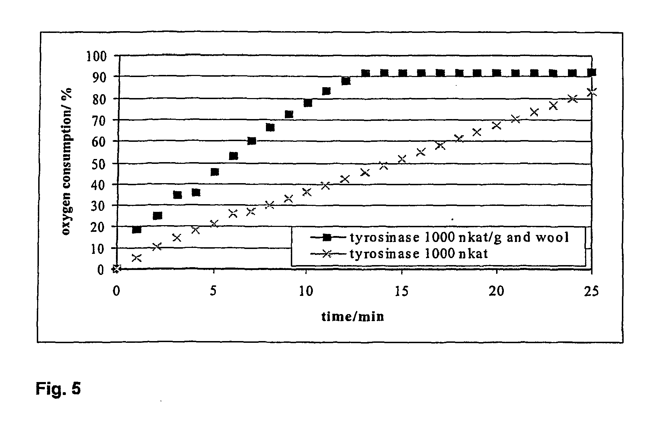

Both potato and novel microbial tyrosinases were able to oxidize wool as measured by oxygen consumption. *Agaricus bisporus*-tyrosinase (Sigma T-7755) was not able to oxidise the tyrosine residues in wool fibres (Figure 4). The tyrosinase produced by the bacterium DSM13540 isolated in this work (Figure 5) consumed significantly more oxygen with wool fibres than the potato tyrosinase (Figure 4).

**Example 9. Effect of enzymatic treatments on wool fibre properties**

Wool fibres (top) were treated with the novel tyrosinase (DSM13540) and potato tyrosinase. The treatments were carried out at 20 rpm agitation in a Linitest Plus machine (Atlas) . The wool to liquid ratio used was 1:15. The different enzyme dosages and the treatment conditions are presented in Table 4. The pH was adjusted with 0.1 M sodium phosphate buffer (pH 7). Reference treatments were carried out similarly but without addition of enzyme. Proteinases were either inhibited or enzyme preparations free of proteinases were used.

Table 4. Treatment conditions for wool fibre treatments. The treatment time was 2 hours.

| Enzyme | Enzyme dosage /g wool fibres | pH | T (°C) |
|---|---|---|---|
| Potato tyrosinase | 50 and 400 nkat | 7 | 30 °C |
| Novel DSM13540 tyrosinase | 150 and 1000 nkat | 7 | 45 °C |

The enzymatic treatments were stopped by immersing the fibres for 5 minutes at 75 °C to pH 3.0 solution adjusted with 75 % acetic acid. Thereafter the fibres were rinsed with tap water for 5 minutes and dried at 50 °C. The fibres were finally conditioned according to the SFS 2600 standard (humidity 65 % $\pm$ 2%, temperature 20 °C $\pm$ 2 °C).

Weight loss of wool fibres was determined by weighing the fibres before and after enzymatic treatments. Alkaline solubility of wool fibres were measured according to the IWTO-4-60 (D) standard. The dry weight of the wool fibres was determined by weighing the sample after drying it to constant weight at 105 °C. Simple wettability test of wool fibres were carried out by measuring the sinking time of fibre snips in deionised water in standard conditions. Approximately 10 mg of standard conditioned fibres were cut to small snips and carefully placed on the surface of 50 ml of deionised water. The sinking time of the fibre snips were recorded. The felt ball density of wool fibres was measured according to the standard IWTO 20-69 (D) (Aachener Filztest).

**Effect of enzymatic treatments on weight loss of wool fibres**

Only modest 1.7...3.0 % weight losses of wool fibres were observed during enzymatic treatments. The enzymatic treatments did not increase the weight loss as compared with the reference (Table 5).

Table 5. The weight losses of wool fibres and proteinase activities of the treatment filtrates of tyrosinase and laccase treatments.

| Enzyme | Enzyme dosage nkat/g wool fibres | Fibre analysis |
|---|---|---|
| | | Weight loss of fibres (%) |
| Reference | - | 2.8 |
| Novel DSM13540 tyrosinase | 150 | 1.7 |
| Novel DSM13540 tyrosinase | 1000 | 2.0 |
| Reference | - | 2.5 |
| potato tyrosinase | 50 | 2.5 |
| potato tyrosinase | 400 | 2.1 |

Effect of enzymatic treatments on crosslinking extent of wool fibres

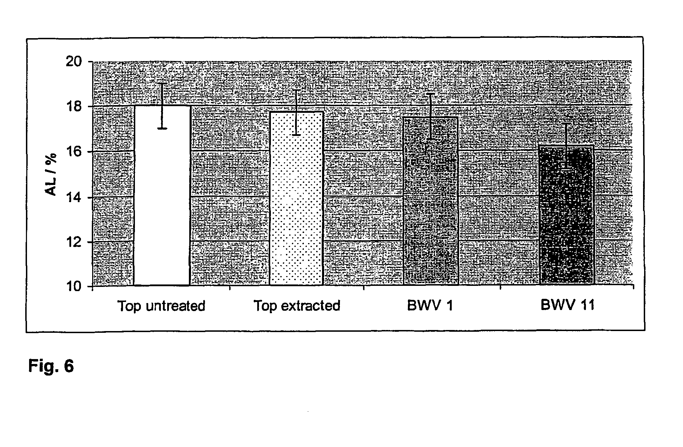

The alkaline solubility was determined according to IWTO 4-60. A mean of 2-3 measurements is given in Figure 6. According to the results the tyrosinase treatment resulted in crosslinking (Fig. 6). BWV 1 is a reference treated in similar conditions but without any enzyme addition.

**Effect of enzymatic treatments on wettability and felting behaviour of wool fibres**

The wettability behaviour of enzymatically treated wool fibres was determined by a simple sinking test. If the enzymatically treated fibres sank faster than the reference treated fibres it was assumed that the hydrophobicity of the fibres had decreased. A decrease in hydrophobicity was observed with potato tyrosinase treated wool fibres.

**Effect of enzymatic treatments on felt ball density**

The wool felting properties were tested by using the Aachen felting test for loose wool (Table 6). According to the results the felting tendency was decreased (Table 6).

Table 6. Felt density of tyrosinase treated wool samples

| Treatment | Felt density/ g/cm3 |
|---|---|
| Untreated | 0.17 |

(continued)

| Treatment | Felt density/ g/cm3 |
|---|---|
| DSM 13540 tyrosinase | 0.14 |

## Effect of enzymatic treatments on chemical composition of wool fibres as analyzed by ESCA and Raman

According to the ESCA analysis a clear oxidation of the wool fibres occurred as indicated by the increase in the amount of oxygen (O) in the tyrosinase treated fibre surfaces and concomitant decrease in the amount of carbon in the surface. Subsequently the O/C ratio was increased which is an indication of oxidation (Table 7)

Table 7. Elemental composition in the surface of enzyme treated BIOWOOL samples (in atom%)

| Code | Enzyme | C | O | N | S | Ca | Na |
|---|---|---|---|---|---|---|---|
| bwv 1 | REF | 79.3 | 10.4 | 7.4 | 2.5 | 0.4 | 0 |
| bwv 11 | DSM TYR 150 | 78.1 | 11 | 8.1 | 2.8 | 0 | 0 |
| bwv 12 | DSM tyr 1000 | 78.2 | 11.6 | 7.6 | 2.7 | 0 | 0 |
| bwv 10 | ref | 80.2 | 10.4 | 6.3 | 2.4 | 0.7 | 0 |
| bwv 13 | Potato tyr 50 | 78.5 | 11.7 | 7.4 | 2.4 | 0 | 0 |
| bwv 14 | Potato tyr 400 | 76.1 | 13.7 | 8.4 | 1.9 | 0 | 0 |

## Raman Spectroscopic Analysis of Enzyme Treated Wool Top

The Raman spectra of proteins exhibit characteristic vibrations of amino acids, in particular at $643cm^{-1}$ the spectral intensity can be associated with tyrosine. In general the Raman data collected shows reasonable reproducibility between comparable samples, treated at different stages of the project and in addition there is evidence for modification of the tyrosine by the enzyme systems, Table 8. Treatment conditions: 0.1M NaP buffer, 30°C, potato tyrosinase pH 7.

Table 8. Raman Intensity Data of Treated Wool Top

| Treatment Conditions | Spectral Intensity at $643cm^{-1}$ |
|---|---|
| Control | 0.2625 |
| Tyr 1000 nkat/g | 0.2098 |
| Tyr 400 nkat/g, pH 7, 30°C | 0.1879 |

## References

Bernan, V., Filpula, D., Herber, W., Bibb, M. and Katz, E. The nucleotide sequence of the tyrosinase gene from Streptomyces antibioticus and characterization of the gene product. Gene 37 (1985) 101-110.

Byrne, K. M., Machine-washable knitwear-Production routes, In: Chemistry of the textiles industry, Ed. C. M. Carr, Chapman & Hall, London, UK, 1995, 187-209.

Dabbous, M. K., Inter- and Intramolecular Cross-Linking in Tyrosinase-treated Tropocollagen, J. Biol. Chem. 22 (1966) 5307-5312.

Ellis, J., Scouring, enzymes and softeners, In: Chemistry of the textiles industry, Ed. C. M. Carr, Chapman & Hall, London, UK, 1995, 249-275.

Fujita, Y., Uraga, Y. and Ichisima, E. Molecular cloning and nucleotide sequence of the protyrosinase gene, melO, from Aspergillus oryzae and expression of the gene in yeast cells. Biochim. Biophys. Acta 1261 (1995) 151-154.

Gaykema, W.P.J., Hol, W. G. J., Vereijken, J. M., Soeter, N. M., Bak, H. J. and Beintema, J.J. 3.2 A structure of the copper-containing, oxygen-carrying protein Panulius interruptus haemocyanin. Stature 309 (1984) 23-29.

Giebel, L., Strunk, K. M. and Spritz, R. A. Organization and nucleotide sequences of the human tyrosinase gene and a truncated tyrosinase-related segment. Genomics 9 (1991) 435-445.

Huber, M., Hintermann, G. and Lerch, K. Primary structure of tyrosinase from Streptomyces glaucescens. Biochemisty 24 (1985) 6038-6044.

Ikeda, K., Masujima, T., Suzuki, K. and Sugiyama, M., Cloning and sequence analysis of the highly expressed melanin-synthesizing gene operon from Streptomyces castaneoglobisporus, Appl. Microbiol. Biotechnol. 45 (1996) 80-85.

Kawamoto, S., Nakamura, M. and Yashima, S. Cloning, sequence and expression of the tyrosinase gene from Streptomyces lavendulae MA406 A-1. J. Ferment. Bioeng., 76 (1993) 345-355.

Kong, K.-H., Hong, M.-P., Choi, S.-S., Kim, Y.-T. and Cho, S.-H., Purification and characterisation of a highly stable tyrosinase from Thermomicrobium roseum, Biotechnol. Appl. Biochem. 31 (2000) 113-118.

Kupper, U., Niedermann, D. M., Travaglini, G. and Lerch, K. Isolation and characterization of the tyrosinase gene from Neurospora crassa. J. Biol. Chem. 264 (1989) 17250-17258.

Kwon, B. S., Haq, A. K., Pomerantz, S. H. and Halaban, R. Isolation and sequence of a cDNA clone for human tyrosinase that maps at the mouse c-albino locus. Proc. Natl. Acad. Sci USA 84 (1987) 7473-7477.

Kwon, B. S., Wakulchik, M., Haq, A. K., Halaban, R. and Kestler, D. Sequence analysis of mouse tyrosinase cDNA and the effect of melanotropin on its gene expression. Biochem. Biophys. Res. Commun. 153 (1988) 1301-1309.

Lerch, K. Copper monooxygenases: tyrosinase and dopamine β-monooxygenase, In Metal Ions in Biological Systems, V ol. 13, Ed. H. Siegel, Marcel Dekker Inc., New York, USA, 1981, 143-186.

Lewis, D. M., Ancillary processes in wool dyeing, In Wool Dyeing, Ed. D. M. Lewis, Society of Dyers and Colorists, West Yorkshire, England, 1992, 111-136.

Makinson, K. R., Shrinkproofing of wool, Marcel Dekker Inc., New York, USA, 1979, 379.

Matheis, G. and Whitaker, J. R., Modification of Proteins by Polyphenol Oxidase and Peroxidase and Their Products, J. Food Biochem. 8 (1984a) 137-162.

Matheis, G. and Whitaker, J. R., Peroxidase-Catalyzed Cross Linking of Proteins, J. Protein Chem. 3 (1984b) 35-48.

Matheis, G. and Whitaker, J. R., A review: Enzymatic Cross-linking of Proteins Appicable to Foods, J. Food Biochem. 11 (1987) 309-327.

Mayer, A. M. and Harel, E., Review: Polyphenol oxidases in plants, Phytochemistry 18 (1979) 193-215.

Mayer, A. M., Polyphenol oxidases in plants - recent progress, Phytochemistry 26 (1987) 11-20.

Mercado-Blanco, J., Garcia, F., Fernandes-Lopez, M. and Olivares, J. Melanin production by Rhizobium meliloti GR4 is linked to non symbiotic plasmid pRmeGR4b: cloning, sequencing, and expression of the tyrosinase gene mepA. J. Bacteriol., 175 (1993) 5403-5410.

Muller, G., Ruppert, S., Schmid, E. and Schutz, G.Functional analysis of alternatively spliced tyrosinase gene transcripts. EMBO J. 7 (1988) 2723-2730.

Paasivirta, L.-L.,Screening of microbes producing phenoloxidases, Master's Thesis, Helsinki University of Technology, Department of Chemical Technology, Espoo, (2000) 87 p.

Takase, M., Miura, I., Nakata, A., Takeuchi, T. and Nishioka, M. Cloning and sequencing of the cDNA encoding tyrosinase of the Japanese pond frog, Rana nigromaculata. Gene 121 (1992) 359-363.

**Claims**

1. A method for enzymatic treatment of a protein structure of proteinaceous fibres, comprising contacting protein fibre, selected from the group of wool, wool fibre, animal hair, silk or spidersilk, with an aqueous solution comprising tyrosinase enzyme under conditions suitable for the function of the enzyme resulting in oxidation of tyrosine residues present in the proteinaceous fibres and crosslinking of the proteins in the proteinaceous fibre.

2. The method according to claim 1, wherein the tyrosinase treatment results in oxidation of tyrosine residues in proteinaceous fibres in either hydroxylation or alternatively both hydroxylation and subsequent quinone formation from these residues.

3. The method according to claim 1, wherein tyrosinase originates from berries, fruits, preferably citrus fruits, such as grape or from apple, pear, peach, banana or from vegetables, such as potato, cabbage, pea, bean, cucumber, tomato, spinach, olive or from cereals, such as barley, wheat, rye or from tea, coffee- and cacao-beans or from animals, such as mouse, frog, shrimps, or from edible mushrooms, such as *Agaricus, Agaricus bisporus, Pleurotus* or *Lentinus.*

4. The method according to claim 1, wherein tyrosinase originates from fungi, such as from *Neurospora crassa, Aspergillus, Aspergillus oryzae, Trametes, Trametes hirsuta, Trametes versicolor* or from *Myceliophthora.*

5. The method according to claim 1, wherein tyrosinase originates from bacteria, such as from *Pseudomonas, P. maltophilia,, Xanthomonas, X maltophilia or Stenotrophomonas, S. maltophilia, Streptomyces, S. glaucescens, S. antibioticus, S. castaneoglobisporus, Vibrio, V. tyrosinaticus, Rosebacterium, Thermomicrobium, T. roseum, Marinomonas, M mediterranea, Alternaria, A. tenuis, Alteromonas* or from *Rhizobium.*

6. The method according to claim 1, wherein tyrosinase is selected from the group comprising tyrosinases from potato or from bacteria belonging to *Pseudomonadaceae,* such as *Pseudomonas sp., Xanthomonas sp.* or *Stenotrophomonas sp..*

7. The method according to any one of the preceding claims, wherein tyrosinase is produced by a recombinant host by expressing a tyrosinase gene encoding the enzyme in an expression or production host

8. The method according to any one of the preceding claims, wherein the proteinaceous material comprises wool, wool fibre or animal hair, such as angora, mohair, cashmere, alpacca, merino and Shetland wool or other commercially useful animal hair product, which originates from, for example, sheep, goat, lama, camel or rabbit, or wherein the proteinaceous material is silk or spidersilk.

9. The method according to any one of the preceding claims, wherein the fibres are in the form of fibre, top, yarn or woven or knitted fabric or garments.

10. The method according to any one of the preceding claims, wherein the treated material comprises blended materials containing at least 20 % of protein fibres.

11. The method according to claim 10, wherein the blended materials comprise cellulosic fibres, such as cotton, linen, ramie, jute, sisal, rayon, acetate, viscose or synthetic fibres such as polyamide, polyester, polypropylene, acrylic, spandex, nylon.

12. The method according to any one of the preceding claims, wherein the amount of tyrosinase is 1 nkat/g - 5000 nkat/g of protein fibre material or 10 mg/kg - 50 g/kg of protein fibre material, preferably 10 nkat/g - 500 nkat/g or 100 mg/kg to 5 g/kg protein fibre material.

13. The method according to any one of the preceding claims, wherein the treatment is carried out at pH range pH 3-8, preferably at pH range 5-7.

14. The method according to any one of the preceding claims, wherein the treatment is carried out in the temperature 20 - 80 °C, preferably in 30-70 °C, most preferably in 40 - 50 °C.

15. The method according to any one of the preceding claims, wherein the treatment is carried out before, during and/or under chemical shrink resist treatment.

16. The method according to any one of the preceding claims, wherein the treatment is carried out before and/ or during dyeing.

17. The method according to any one of the preceding claims, wherein the treatment is carried out before and/ or during any finishing treatment.

18. The method according to any one of the preceding claims, wherein the tyrosinase treatment is combined with an reducing agent, such as ascorbic acid.

19. The method according to any one of the preceding claims, wherein proteinaceous fibres are treated before, during or after the tyrosinase treatment with another enzymatic or a chemical conventional shrink reduction treatment.

20. The method according to claim 19, wherein the other enzyme is lipase, lipoxygenase, transglutaminase, laccase, protease, peroxidase, haloperoxidase, protein disulphide isomerase or any of their combinations.

21. The method according to any one of the preceding claims, wherein the treatment comprises agitation.

22. The method according to any one of the preceding claims, wherein the treatment is carried out without agitation.

23. Proteinaceous fibres selected from the group of wool, wool fibre, animal hair, silk or spidersilk treated according to the method of any one of the preceding claims.

24. The fibres according to claim 23, which comprise fabric, garment, fibre, top or animal hair.

**Patentansprüche**

1. Ein Verfahren zur enzymatischen Behandlung einer Proteinstruktur proteinartiger Fasern, umfassend das in Kontakt bringen von Proteinfaser, ausgewählt aus der Gruppe aus Wolle, Wollfaser, Tierhaar, Seide oder Spinnenseide, mit einer wässrigen Lösung, umfassend Tyrosinase-Enzym, unter Bedingungen, die für die Funktionsweise des Enzyms geeignet sind, was eine Oxidation von Tyrosin-Resten, die in den proteinartigen Fasern vorhanden sind, und ein Vernetzen der Proteine in der proteinartigen Faser zur Folge hat.

2. Das Verfahren nach Anspruch 1, wobei die Tyrosinasebehandlung eine Oxidation von Tyrosin-Resten in proteinartigen Fasern entweder durch Hydroxylierung oder alternativ sowohl durch Hydroxylierung als auch anschließender Chinonbildung aus diesen Resten zur Folge hat.

3. Das Verfahren nach Anspruch 1, wobei Tyrosinase aus Beeren, Früchten, vorzugsweise Zitrusfrüchten, wie z.B. Traube, oder aus Apfel, Birne, Pfirsich, Banane, oder aus Gemüse, wie z.B. Kartoffel, Kohl, Erbse, Bohne, Gurke, Tomate, Spinat, Olive, oder aus Getreide, wie z.B. Gerste, Weizen, Roggen, oder aus Tee, Kaffee- und Kakaobohnen oder aus Tieren, wie z.B. Maus, Frosch, Garnelen, oder aus essbaren Pilzen, wie z.B. *Agaricus, Agaricus bisporus, Pleurotus* oder *Lentinus,* stammt.

4. Das Verfahren nach Anspruch 1, wobei Tyrosinase aus Pilzen, wie z.B. aus *Neurospora crassa, Aspergillus, Aspergillus oryzae, Trametes, Trametes hirsuta, Trametes versicolor* oder aus *Myceliophthora* stammt.

5. Das Verfahren nach Anspruch 1, wobei Tyrosinase aus Bakterien, wie z.B. aus *Pseudomonas, P. maltophilia, Xanthomonas, X. maltophilia* oder *Stenotrophomonas, S. maltophilia, Streptomyces, S. glaucescens, S. antibioticus, S. castaneoglobisporus, Vibrio, V. tyrosinaticus, Rosebacterium, Thermomicrobium, T. roseum, Marinomonas, M. mediterranea, Alternaria, A. tenuis, Alteromonas* oder aus *Rhizobium* stammt.

6. Das Verfahren nach Anspruch 1, wobei Tyrosinase aus der Gruppe umfassend Tyrosinasen aus Kartoffel oder aus Bakterien, die zu *Pseudomonadaceae* gehören, wie z.B. *Pseudomonas sp., Xanthomonas sp.* oder *Stenotrophomo-*

*nas sp.,* ausgewählt ist.

7. Das Verfahren nach einem der vorhergehenden Ansprüche, wobei Tyrosinase durch einen rekombinanten Wirt durch Expression eines für das Enzym kodierenden Tyrosinase-Gens in einem Expressions- oder Produktionswirt produziert wird.

8. Das Verfahren nach einem der vorhergehenden Ansprüche, wobei das proteinartige Material Wolle, Wollfaser oder Tierhaar, wie z.B. Angora-, Mohair-, Kaschmir-, Alpaka-, Merino- und Shetland-Wolle oder ein anderes Tierhaarprodukt von kommerziellem Nutzen umfasst, das z.B. von Schaf, Ziege, Lama, Kamel oder Hase stammt, oder wobei das proteinartige Material Seide oder Spinnenseide ist.

9. Das Verfahren nach einem der vorhergehenden Ansprüche, wobei die Fasern in der Form von Faser, Top, Garn oder Gewebe oder Maschenware oder Kleidungsstücken vorliegen.

10. Das Verfahren nach einem der vorhergehenden Ansprüche, wobei das behandelte Material Mischmaterialien umfasst, die mindestens 20 % Proteinfasern enthalten.

11. Das Verfahren nach Anspruch 10, wobei die Mischmaterialien Celluosefasern, wie z.B. Baumwoll-, Leinen-, Ramie-, Jute-, Sisal-, Reyon-, Acetat-, Viskose- oder synthetische Fasern, wie z.B. Polyamid, Polyester, Polypropylen, Acryl, Spandex, Nylon, umfassen.

12. Das Verfahren nach einem der vorhergehenden Ansprüche, wobei die Menge an Tyrosinase 1 nkat/g - 5000 nkat/g Proteinfasermaterial oder 10 mg/kg - 50 g/kg Proteinfasermaterial, vorzugsweise 10 nkat/g - 500 nkat/g oder 100 mg/kg bis 5 g/kg Proteinfasermaterial beträgt.

13. Das Verfahren nach einem der vorhergehenden Ansprüche, wobei die Behandlung bei einem pH-Bereich von pH 3-8, vorzugsweise bei einem pH-Bereich von 5-7 durchgeführt wird.

14. Das Verfahren nach einem der vorhergehenden Ansprüche, wobei die Behandlung bei der Temperatur von 20 - 80°C, vorzugweise bei 30 - 70°C, am meisten bevorzugt bei 40 - 50°C durchgeführt wird.

15. Das Verfahren nach einem der vorhergehenden Ansprüche, wobei die Behandlung vor, während und/oder unter chemischer schrumpffest-machender Behandlung durchgeführt wird.

16. Das Verfahren nach einem der vorhergehenden Ansprüche, wobei die Behandlung vor und/oder während des Färbens durchgeführt wird.

17. Das Verfahren nach einem der vorhergehenden Ansprüche, wobei die Behandlung vor und/oder während einer Appreturbehandlung durchgeführt wird.

18. Das Verfahren nach einem der vorhergehenden Ansprüche, wobei die Tyrosinasebehandlung mit einem Reduktionsmittel, wie z.B. Ascorbinsäure, kombiniert wird.

19. Das Verfahren nach einem der vorhergehenden Ansprüche, wobei proteinartige Fasern vor, während oder nach der Tyrosinasebehandlung mit einer anderen enzymatischen oder einer chemischen herkömmlichen, das Schrumpfen verringernden Behandlung behandelt werden.

20. Das Verfahren nach Anspruch 19, wobei das andere Enzym Lipase, Lipoxygenase, Transglutaminase, Laccase, Protease, Peroxidase, Haloperoxidase, Proteindisulfidisomerase oder eine ihrer Kombinationen ist.

21. Das Verfahren nach einem der vorhergehenden Ansprüche, wobei die Behandlung Rühren umfasst.

22. Das Verfahren nach einem der vorhergehenden Ansprüche, wobei die Behandlung ohne Rühren durchgeführt wird.

23. Proteinartige Fasern, ausgewählt aus der Gruppe aus Wolle, Wollfaser, Tierhaar, Seide oder Spinnenseide, behandelt gemäß dem Verfahren nach einem der vorhergehenden Ansprüche.

24. Die Fasern nach Anspruch 23, welche Gewebe, Kleidungsstücke, Faser, Top oder Tierhaar umfassen.

**Revendications**

1. Procédé de traitement enzymatique d'une structure protéique de fibres protéiques comprenant la mise en contact de fibres protéiques, choisies dans le groupe comprenant la laine, les fibres de laine, les poils d'animaux, la soie ou la soie d'araignée, avec une solution aqueuse comprenant l'enzyme tyrosinase dans des conditions appropriées pour la fonction de l'enzyme entraînant l'oxydation de résidus de tyrosine présents dans les fibres protéiques et la réticulation des protéines dans la fibre protéique.

2. Procédé selon la revendication 1, dans lequel le traitement par tyrosinase entraîne l'oxydation des résidus de tyrosine dans les fibres protéiques en hydroxylation ou alternativement, à la fois hydroxylation et formation consécutive de quinone à partir de ces résidus.

3. Procédé selon la revendication 1, dans lequel la tyrosinase provient de baies, de fruits, de préférences des agrumes, par exemple des raisins ou des pommes, des poires, pêches, bananes ou de légumes tels que pomme de terre, chou, pois, haricot, concombre, tomate, épinard, olive ou de céréales telles que orge, blé, seigle ou de thé, de fèves de café ou de cacao ou d'animaux tels que souris, grenouille, crevettes, ou de champignons comestibles tels que *Agaricus, Agaricus bisporus, Pleurotus* ou *Lentinus.*

4. Procédé selon la revendication 1, dans lequel la tyrosinase provient de champignons tels que *Neurospora crassa, Aspergillus, Aspergillus oryzae, Trametes, Trametes hirsuta, Trametes versicolor* ou de *Myceliophthora.*

5. Procédé selon la revendication 1, dans lequel la tyrosinase provient de bactéries telles que *Pseudomonas, P. maltophilia, Xanthomonas, X. maltophilia ou Stenotrophomonas, S. maltophilia, Streptomyces, S. glaucescens, S. antibioticus, S. castaneoglobisporus, Vibrio, V. tyrosinaticus, Rosebacterium, Thermomicrobium, T. raseum, Marinomonas, M. mediterranea, Alternaria* ou de *Rhizobium.*

6. Procédé selon la revendication 1, dans lequel la tyrosinase est choisie dans le groupe comprenant la tyrosinase de pomme de terre ou de bactéries appartenant aux *Pseudomonadaceae* telles que *Pseudomonas sp., Xanthomonas sp.* ou *Stenotrophomonas sp.*

7. Procédé selon l'une quelconque des revendications précédentes, dans lequel la tyrosinase est produite par un hôte recombinant en exprimant un gène de la tyrosinase codant pour l'enzyme dans un hôte d'expression ou de production.

8. Procédé selon l'une quelconque des revendications précédentes, dans lequel le matériau protéique comprend la laine, les fibres de laine ou les poils d'animaux tels que les laines angora, mohair, cashmere, alpaga, mérinos ou shetland ou autres produits de poils d'animaux utiles commercialement, qui proviennent par exemple de moutons, de chèvres, de lamas, de chameaux ou de lapins, ou dans lequel le matériau protéique est la soie ou la soie d'araignée.

9. Procédé selon l'une quelconque des revendications précédentes, dans lequel les fibres se présentent sous la forme de fibres, de rubans de laine peignée, de fils ou de tissus ou vêtements tissés ou tricotés.

10. Procédé selon l'une quelconque des revendications précédentes, dans lequel le matériau traité comprend des matériaux mélangés contenant au moins 20 % de fibres protéiques.

11. Procédé selon la revendication 10, dans lequel les matériaux mélangés comprennent des fibres cellulosiques telles que le coton, le lin, la ramie, le jute, le sisal, la rayonne, l'acétate, la viscose ou des fibres synthétiques telles que polyamide, polyester, polypropylène, acrylique, élasthanne, nylon.

12. Procédé selon l'une quelconque des revendications précédentes, dans lequel la quantité de tyrosinase est de 1 nkat/g à 5000 nkat/g de matériau de fibres protéiques ou 10 mg/kg à 50 g/kg de matériau de fibres protéiques, de préférence, 10 nkat/g à 500 nkat/g ou 100 mg/kg à 5 g/kg de matériau de fibres protéiques.

13. Procédé selon l'une quelconque des revendications précédentes, dans lequel le traitement est réalisé dans une plage de pH de 3 à 8, de préférence dans une plage de pH de 5 à 7.

14. Procédé selon l'une quelconque des revendications précédentes, dans lequel le traitement est réalisé à la température de 20 à 80° C, de préférence, de 30 à 70° C, de manière préférée entre toutes, de 40 à 50° C.

**15.** Procédé selon l'une quelconque des revendications précédentes, dans lequel le traitement est réalisé avant, pendant et/ou sous le traitement chimique de résistance au rétrécissement.

**16.** Procédé selon l'une quelconque des revendications précédentes, dans lequel le traitement est réalisé avant et/ou pendant la teinture.

**17.** Procédé selon l'une quelconque des revendications précédentes, dans lequel le traitement est réalisé avant et/ou pendant un traitement de finissage.

**18.** Procédé selon l'une quelconque des revendications précédentes, dans lequel le traitement par tyrosinase est combiné à un agent réducteur tel que l'acide ascorbique.

**19.** Procédé selon l'une quelconque des revendications précédentes, dans lequel les fibres protéiques sont traitées avant, pendant ou après le traitement par tyrosinase avec un autre traitement enzymatique ou un traitement chimique classique de résistance au rétrécissement.

**20.** Procédé selon la revendication 19, dans lequel l'autre enzyme est la lipase, la lipoxygénase, la transglutaminase, la lactase, la protéase, la peroxydase, l'haloperoxydase, la disulfure isomérase protéique ou l'une quelconque de leurs combinaisons.

**21.** Procédé selon l'une quelconque des revendications précédentes, dans lequel le traitement comprend une agitation.

**22.** Procédé selon l'une quelconque des revendications précédentes, dans lequel le traitement est réalisé sans agitation.

**23.** Fibres protéiques choisies dans le groupe comprenant la laine, les fibres de laine, les poils d'animaux, la soie ou la soie d'araignée traitées selon le procédé selon l'une quelconque des revendications précédentes.

**24.** Fibres selon la revendication 23, qui comprend les tissus, vêtements, fibres, rubans de laine peignée ou poils d'animaux.

**Fig. 1**

**Fig. 2**

Fig. 3A

Fig. 3B

Fig. 4

**Fig. 5**

**Fig. 6**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 618986 A
- WO 9619611 A
- WO 9827264 A
- US 5529928 A
- EP 358386 A
- EP 134267 A
- WO 9960200 A

- WO 9425574 A
- WO 9805816 A
- WO 0031333 A
- WO 9915137 A
- US 6140109 A
- EP 947142 B
- FI 20001808

**Non-patent literature cited in the description**

- **Bernan, V. ; Filpula, D. ; Herber, W. ; Bibb, M. ; Katz, E.** The nucleotide sequence of the tyrosinase gene from Streptomyces antibioticus and characterization of the gene product. *Gene,* 1985, vol. 37, 101-110
- Machine-washable knitwear-Production routes. **Byrne, K. M.** Chemistry of the textiles industry. Chapman & Hall, 1995, 187-209
- **Dabbous, M. K.** Inter- and Intramolecular Cross-Linking in Tyrosinase-treated Tropocollagen. *J. Biol. Chem.,* 1966, vol. 22, 5307-5312
- Scouring, enzymes and softeners. **Ellis, J.** Chemistry of the textiles industry. Chapman & Hall, 1995, 249-275
- **Fujita, Y. ; Uraga, Y. ; Ichisima, E.** Molecular cloning and nucleotide sequence of the protyrosinase gene, melO, from Aspergillus oryzae and expression of the gene in yeast cells. *Biochim. Biophys. Acta,* 1995, vol. 1261, 151-154
- **Gaykema, W.P.J. ; Hol, W. G. J. ; Vereijken, J. M. ; Soeter, N. M. ; Bak, H. J. ; Beintema, J.J. 3.2.** A structure of the copper-containing, oxygen-carrying protein Panulius interruptus haemocyanin. *Stature,* 1984, vol. 309, 23-29
- **Giebel, L. ; Strunk, K. M. ; Spritz, R. A.** Organization and nucleotide sequences of the human tyrosinase gene and a truncated tyrosinase-related segment. *Genomics,* 1991, vol. 9, 435-445
- **Huber, M. ; Hintermann, G. ; Lerch, K.** Primary structure of tyrosinase from Streptomyces glaucescens. *Biochemisty,* 1985, vol. 24, 6038-6044
- **Ikeda, K. ; Masujima, T. ; Suzuki, K. ; Sugiyama, M.** Cloning and sequence analysis of the highly expressed melanin-synthesizing gene operon from Streptomyces castaneoglobisporus. *Appl. Microbiol. Biotechnol.,* 1996, vol. 45, 80-85

- **Kawamoto, S. ; Nakamura, M. ; Yashima, S. Cloning.** sequence and expression of the tyrosinase gene from Streptomyces lavendulae MA406 A-1. *J. Ferment. Bioeng.,* 1993, vol. 76, 345-355
- **Kong, K.-H. ; Hong, M.-P. ; Choi, S.-S. ; Kim, Y.-T. ; Cho, S.-H.** Purification and characterisation of a highly stable tyrosinase from Thermomicrobium roseum. *Biotechnol. Appl. Biochem.,* 2000, vol. 31, 113-118
- **Kupper, U. ; Niedermann, D. M. ; Travaglini, G. ; Lerch, K.** Isolation and characterization of the tyrosinase gene from Neurospora crassa. *J. Biol. Chem.,* 1989, vol. 264, 17250-17258
- **Kwon, B. S. ; Haq, A. K. ; Pomerantz, S. H. ; Halaban, R.** Isolation and sequence of a cDNA clone for human tyrosinase that maps at the mouse c-albino locus. *Proc. Natl. Acad. Sci USA,* 1987, vol. 84, 7473-7477
- **Kwon, B. S. ; Wakulchik, M. ; Haq, A. K. ; Halaban, R. ; Kestler, D.** Sequence analysis of mouse tyrosinase cDNA and the effect of melanotropin on its gene expression. *Biochem. Biophys. Res. Commun.,* 1988, vol. 153, 1301-1309
- Copper monooxygenases: tyrosinase and dopamine β-monooxygenase. **Lerch, K.** Metal Ions in Biological Systems. Marcel Dekker Inc, 1981, vol. 13, 143-186
- Ancillary processes in wool dyeing. **Lewis, D. M.** Wool Dyeing. Society of Dyers and Colorists, 1992, 111-136
- **Makinson, K. R.** Shrinkproofing of wool. Marcel Dekker Inc, 1979, 379
- **Matheis, G. ; Whitaker, J. R.** Modification of Proteins by Polyphenol Oxidase and Peroxidase and Their Products. *J. Food Biochem.,* 1984, vol. 8, 137-162

- **Matheis, G. ; Whitaker, J. R.** Peroxidase-Catalyzed Cross Linking of Proteins. *J. Protein Chem.,* 1984, vol. 3, 35-48
- **Matheis, G. ; Whitaker, J. R.** A review: Enzymatic Cross-linking of Proteins Appicable to Foods. *J. Food Biochem.,* 1987, vol. 11, 309-327
- **Mayer, A. M. ; Harel, E.** Review: Polyphenol oxidases in plants. *Phytochemistry,* 1979, vol. 18, 193-215
- **Mayer, A. M.** Polyphenol oxidases in plants - recent progress. *Phytochemistry,* 19870000, vol. 26, 11-20
- **Mercado-Blanco, J. ; Garcia, F. ; Fernandes-Lopez, M. ; Olivares, J.** Melanin production by Rhizobium meliloti GR4 is linked to non symbiotic plasmid pRmeGR4b: cloning, sequencing, and expression of the tyrosinase gene mepA. *J. Bacteriol.,* 1993, vol. 175, 5403-5410
- **Muller, G. ; Ruppert, S. ; Schmid, E. ; Schutz, G.** Functional analysis of alternatively spliced tyrosinase gene transcripts. *EMBO J.,* 1988, vol. 7, 2723-2730
- **Paasivirta, L.-L.** Screening of microbes producing phenoloxidases. *Master's Thesis,* 2000, 87
- **Takase, M. ; Miura, I. ; Nakata, A. ; Takeuchi, T. ; Nishioka, M.** Cloning and sequencing of the cDNA encoding tyrosinase of the Japanese pond frog. *Rana nigromaculata. Gene,* 1992, vol. 121, 359-363